# EUROPEAN PATENT SPECIFICATION

(11) **EP 2 211 824 B1**
(45) Date of publication and mention of the grant of the patent: **02.09.2015**
(21) Application number: 08838662.8
(22) Date of filing: 20.10.2008
(51) Int. Cl.: A61K 8/19, A61K 8/36, A61K 8/04, A61Q 9/02, A61Q 19/00, A61K 8/365

(54) **SHAVING COMPOSITION WITH SKINCARE PROPERTIES**
RASIERZUSAMMENSETZUNG MIT AFTERSHAVE-HAUTPFLEGEEIGENSCHAFT
COMPOSITION DE RASAGE AVEC SOIN APRÈS-RASAGE

(30) Priority: 18.10.2007 EP 07118819
(43) Date of publication of application: 04.08.2010
(73) Proprietor: Unilever PLC, London EC4Y 0DY (GB); Unilever N.V., 3013 AL Rotterdam (NL)
(72) Inventor: KLOMP, Andreas Joannes Anthonius, NL-2285 JE Rijswijk (NL); PRONK, Johannes, NL-2725 AM Zoetermeer (NL); BUITELAAR, Thomas Andreas, NL-3155 VC Maasland (NL); van de Veer-de Nijs, Willemijn, NL-2497 AK Den Haag (NL)
(74) Representative: Acham, Nicholas Clive
(86) International application number: PCT/NL2008/050658
(87) International publication number: WO 2009/051486

(56) References cited:
- EP-A- 0 034 126
- WO-A-01/85129
- WO-A-94/26233
- FR-A- 2 635 682
- US-A- 5 626 852

## Description

### Field of the Invention

The invention is in the field of essentially soap-free shaving compositions. The invention further relates to the use of an emulsion composition in the manufacture of a shaving skin care product, particularly in the preparation and the execution of a wet shave and in reducing and/or repairing skin damage resulting from mechanical and/or chemical processes such as shaving.

### Background of the Invention

A variety of shaving compositions exists, for use in mechanical or electrical wet-shave. Shaving compositions generally serve to soften the hair prior to being cut, by the application of wetted soap lathers, foams, such as instant foams, gels, such as post-foaming gels, oils, and the like. Most of these require separate aftershave skin care in order to calm or condition the skin after shaving.

As they are marketed, some of these compositions are directed to both functions, i.e. the facilitation of hair-removal and the aftershave skin care.

A reference in this respect is, for example, Shave & Refresh Shaving Gel from King of Shaves Woman, which delivers a comfortable shave and moisturisation of the skin. Similar systems are available and comprises a polymer thickened gel system using synthetic (co)polymers (e.g. acrylates/C10-30 alkyl acrylate crosspolymer) or other gelling agents.

The combination of shave and aftershave properties has received attention also in different solutions, such as disclosed in US2003/143253 A1. A combination of stearic acid and glycerine, or a comparable ingredient, serves to reduce the undesired skin-damaging effects of shaving.

It is well-appreciated that skin shaving brings about the removal of several layers of skin, resulting in loss of skin barrier function which leads to drying out of the skin and to less resistance to colonization and easier penetration of potentially harmful micro-organisms. Hence, from medical, cosmetic as well as hygienic perspective, a rapid recovery of the barrier function of the skin is required. It is further observed that the skin needs to have proper moisture content in order to have an adequate barrier function.

As a further background, US 5,500,211 is referred to. This relates to a soap-free, self-foaming shave gel comprising water, a water-soluble N-acyl sarcosinate salt, a volatile mixture of hydrocarbons, and a non-volatile paraffinic hydrocarbon fluid. The acyl sarcosinate is neutralized with a base, preferably triethanolamine. The resulting pH of the composition is in a range of from 4 to 8.

Another background reference on soap-free shaving compositions is EP 034 126. These compositions essentially include a cationic compound containing at least one quaternary ammonium nitrogen, and have a pH in the range of from 2 to 7.

### Summary of the Invention

It would be advantageous to provide a shaving product which not only aids in the shaving process but, as a particular type of skin care product also contributes to the faster recovery of the skin barrier function after mechanical or chemical damage caused by for example shaving.

In order to better address these desires, the invention, in one aspect, presents use of an essentially soap-free shaving composition in shaving the skin comprising 50-90 wt.% water, 1-15 wt.% humectant, 0.1 to 20 wt.% of a surfactant and/or foam stabilizer, up to 5 wt.% of a structurant such as a thickener, a viscosity enhancer or a rheology modifier and 0.01-6 wt.% of a volatile nitrogen base, the percentages being by weight of the entire composition according to claim 1.

In another aspect, the invention is a shaving composition according to claim 13 or 14.

In yet another aspect, the invention is in the use of an essentially soap-free emulsion comprising the aforementioned components, for the manufacture of a two-in-one shaving and aftershave composition.

### Detailed description of the invention

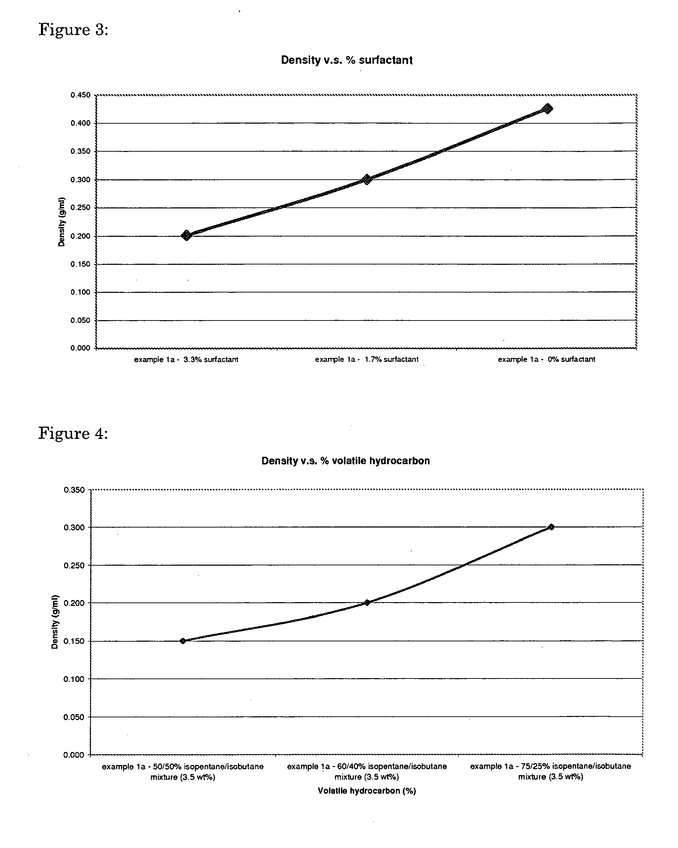

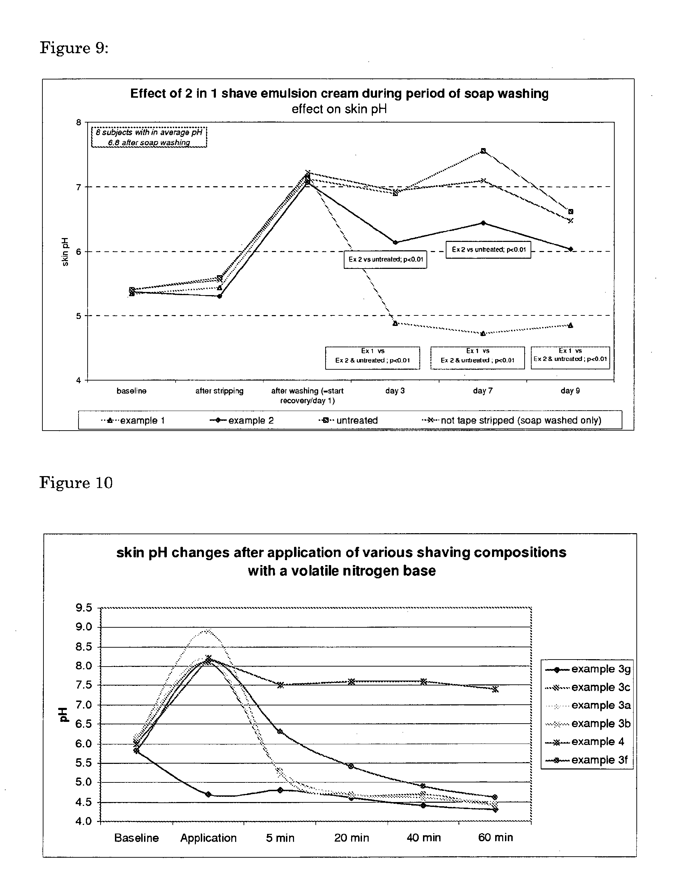

In general, the invention provides a shaving composition, preferably an emulsion composition, including a volatile nitrogen base, that is essentially soap free. The term "essentially soap free" is used to indicate that the composition has maximally 2 wt.% of soap. When applied in a concentration of up to 2 wt.%, and particularly if the concentration is maximally 1 wt. %, this negative effect is considered to be outbalanced by it beneficial effect, such as its foam stabilizing properties. For example, in such a low-soap composition, the soap may be prepared *in situ* by reacting a basic material such as triethanolamine or sodium hydroxide with higher fatty acids, such a stearic, palmitic, myristic, oleic or coconut oil fatty acid. Preferably an excess of alkaline material may be added to completely neutralize the fatty acid and to adjust the pH to the desired value (see Figure 10, 3f).

For comparison, shave foam and shave gel aerosols currently in the market contain soap concentrations of from 8 to 20 wt%.

The volatile nitrogen base is comprised in the composition of the invention to boost the skin repair properties of the composition. After application of the composition to skin, and due to the evaporation of the volatile nitrogen base and water, the pH of the skin will have a value in the range of from 4.0 to 5.0, preferably between 4.2 and 4.9. This pH range will facilitate an optimal recovery of the skin barrier function. In other words, the emulsion composition according to the present invention brings advantageously the pH of the skin to a desired value. The volatile nitrogen base serves as a skin pH control agent and brings the skin pH to the desired value within 5-10 minutes after application.

The shaving compositions according to the present invention have an attractive and beneficial effect on the barrier function of the skin in one or more ways. In particular, the preferred emulsion composition has been found to have a strengthening effect on at least one of the following functions:
(i) the physico-chemical barrier realised by the keratinocytes and the epidermal lipids forming bi-layered sheets embedding those keratinocytes typically found in the deeper layers of the Stratum Corneum,
(ii) the microbiological barrier (at the surface of the Stratum Corneum), and
(iii) proper homeostasis in order to maintain a strong, smooth, unbroken and flexible skin condition (in particular in the superficial layers of the Stratum Corneum).

In the skin, a pH gradient exists from the skin surface to the deepest layers. The pH at the surface is almost always lower than in the deeper parts (such as Stratum Compactum and Stratum Granulosum) where pH reaches that of the internal body fluids.

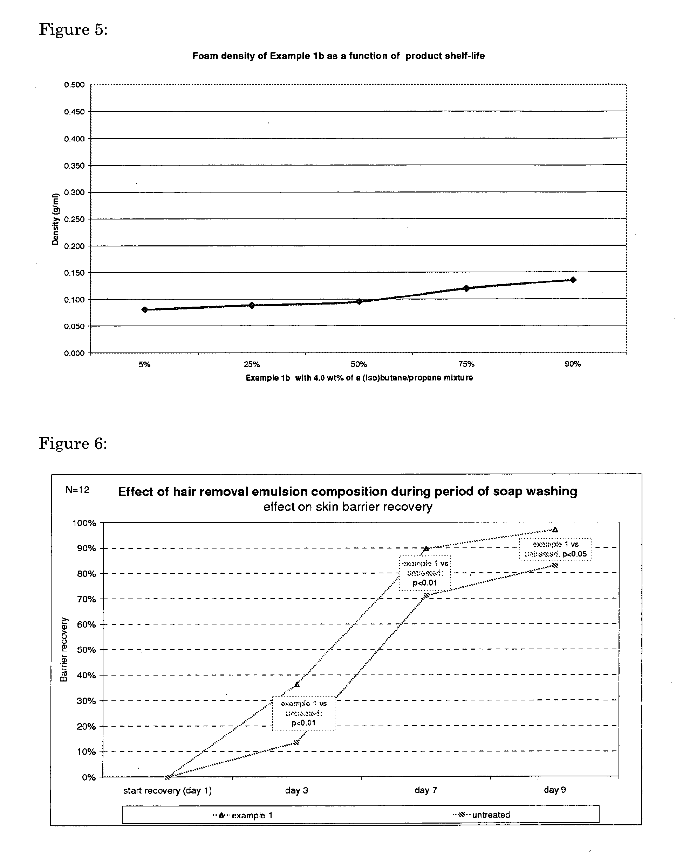

Without wishing to be bound by any theory, the present inventors believe that the positive effect of the shaving composition of the invention on the barrier function of the skin, is partially due to the capacity to restore and/or maintain the pH of general layers of the skin at a neutral (i.e. non-pathological) value. The composition according to the invention thus has been found to have a balancing effect on the skin, which helps to maintain the skin integrity (see Figures 6-9).

The essentially soap-free composition of the invention can generally be a lotion (such as a pre-electric shave lotion), a polymer-thickened gel system or an emulsion.

The invention preferably is a shaving composition in the form of an essentially soap free emulsion. The various ingredients of the compositions are discussed hereinafter. Percentages indicated, are weight percentages based on the total weight of the composition.

Shaving compositions of various kinds have in common that they generally serve to support the razor blade in removing hair from the skin. To this end, the shaving composition comprises one or more ingredients to obtain a twofold action, viz. to soften the hair (water, surfactants/soap), and to act as a lubricant (hydrocarbon oils, silicon oils) between the shaving hardware and the face. For the one or more ingredients that can exercise these functions, in various types of shaving compositions for wet shave or for use in electrical shave, reference is made to Rieger M.M. (Ed.), Harry's Cosmeticology, Chemical Publishing Co. Inc., New York, 501-522 (2000).

Shaving compositions can be in the form of conventional brushless shave creams, which are conventionally based on soap and do not deliver the skin care benefits (skin repair and long lasting moisturisation) as described in this invention.

The effects of the shaving composition in this invention can best be realized when use is made of a particular combination of emulsion components. Accordingly, in a preferred embodiment, the present invention relates to the use, in a shaving composition with skincare properties, of a composition having a pH value in the range of from 4 to 12, which composition comprises 50 to 90 wt.% water, a mixture of hydrocarbon oils, and silicon oils in an amount of 3 to 20 wt.%, a volatile nitrogen base or salt thereof in an amount of 0.01 to 6.0 wt.%, a surfactant (foaming agent) and/or foam stabilizer in an amount of maximally 20 wt.%, a structurant, which could be either a thickener, a viscosity modifier or a consistency enhancere or a combination of in an amount of maximally 5 wt.%, preferably maximally 15 wt. %, a fragrance in an amount of maximally 1.5 wt.%, a humectant in an amount of 1 to 15 wt.%, preferably maximally 10 wt.%, an astringent additive in an amount of maximally 5 wt.%, an bioactive additive in an amount of maximally 5 wt.%, a buffer or base in an amount of maximally 5 wt.% and a volatile hydrocarbon propellant in an amount of maximally 5 wt.%, whereby all weights are based on total emulsion composition. Finally, the shaving composition in this invention effectively controls skin pH and is in essence free of soap.

The amount of water in the preferred emulsion composition according to the present invention can suitably be in the range of from 50 to 90 wt.%, and is preferably in the range of from 65 to75 wt.%, based on total emulsion composition.

The preferred emulsion composition of the invention comprises 3 to 20 wt.% of a mixture of hydrocarbon oils, vegetable oils, synthetic oils and silicon oils. Preferably, a mixture of hydrocarbon and silicon oils is present in an amount in the range of from 5 to 15 wt.% based on total emulsion composition. The oil-mixture preferably contains the hydrocarbon oil in a percentage of from 45 to 95 wt.%, and preferably of from 60 to 85 wt.% and the silicon oil in a percentage of from 5 to 55 wt.%, preferably of from 15 to 40 wt.% The oil is preferably selected from the group of silicon oils such as cyclopentasiloxane, and the group hydrocarbon oils or mineral oils such as paraffin liquidum and (branched) paraffins, such as petrolatum and isohexadecan.

The nitrogen base to be used is a volatile nitrogen base. In the context of the present invention this means that the nitrogen base has a boiling point below 200°C, preferably below 100°C, and more preferably below 65°C. The volatile nitrogen base is preferably selected from the group consisting of ammonia, mono-ethanol amine, alkyl amines of which the alkyl group comprises 1-4 carbon atoms, and mixtures thereof. More preferably, the volatile nitrogen base is ammonia.

In accordance with the present invention the volatile nitrogen base can be present in the form of a salt. Suitable examples of such salts include, for instance, ammonium chloride, ammonium sulphate and ammonium lactate.

The volatile nitrogen base or its salt can suitably be present in an amount of from 0.01-6 wt.% of the total emulsion composition. Preferably, the volatile nitrogen base or its salt is present in an amount of 0.01-4 wt.% and, more preferably 0.5-3 wt.%

The emulsion composition of the invention preferably contains one or more emulsifiers and/or surfactants and/or foam stabilizers in an amount of maximally 20 wt%. Various of emulsifiers, surfactants and or foam stabilizers can be used, however one skilled in the art can easily find alternatives and the following list illustrate some possibilities. Examples of emulsifiers which can be used are ceteareth-20, ceteareth-12, cetyl hydroxyethylcellulose, hexyldecyl palmitate, cetyl palmitate, pentaerythrityl distearate, sodium cetearyl sulfate, glyceryl monostearate, cetearyl glucoside. In addition, either anionic, cationic or non-ionic surfactants or combination of can be used as foaming agents and examples are sodium laureth sulphate, cocoamidopropyl betaine, cocoglucoside, sodium cocyl isehionate. For stabilizing the generated foam, foam stabilizers can be used consisting from the group of fatty alcohols (e.g. cetyl/stearyl-alcohol, cetearylalcohol), and various types of proteins.

A preferred emulsion composition comprises maximally up to 12 wt.% of surfactants. More preferably the system comprises maximally up to 8 wt.% of surfactants.

The structurant (thickener/viscosity enhancer/rheology modifier) or mixture of stucturants to be used in accordance with the present invention is present in an amount of maximally 5 wt.%, based on total emulsion composition. The thickener/viscosity enhancer/rheology modifier to be used can suitably chosen from the group consisting cellulose polymers (e.g. hydroxyethylcellulose, hydroxypropylcellulose), polysaccharide guar gums (e.g. xanthum gum, carrageenan gum, guar gum), or also synthetic (co)polymers (e.g. acrylates/C10-30 alkyl acrylate crosspolymer, carbomers), and or mixtures of the various polymers and a suitable rheology modifier is for example PEG 150 distearate.

In addition, the compositions of the invention can suitably comprise a fragrance. Any fragrance known to be used in body applications or aftershaves can be used for this purpose. The fragrance to be used is suitably present in an amount of maximally 1.5 wt.% based on total emulsion composition.

The compositions in accordance with the present invention further comprise a humectant for promoting retention of moisture of the skin. The humectant to be used in the present compositions can suitably be selected from the group consisting of glycols, amino acids, hydroxylated compounds, urea, pyrrolidine carboxylic acid, and any combinations thereof. Examples of suitable humectants include glycerol and sorbitol. Preferably, the humectant to be used in accordance with the present invention is glycerol or sorbitol and or mixtures of both. Suitably, the humectant is present in an amount in the range of from 1 to 15 wt.%, based on total composition. Preferably, the humectant is present in an amount in the range of from 4 to 10 wt.%, based on total composition.

Suitably, the present emulsion composition comprises an astringent additive. Such an astringent additive tightens the skin. Suitably, the astringent additive is selected from the group consisting of metal salts, tannins and hazel extracts. Preferably, the astringent additive is a soluble aluminium salt or a soluble zinc salt. More preferably, the astringent additive is, alum or zinc sulphate. The astringent additive is suitably present up to an amount of 5 wt.%, based on total composition, preferably 0.3 to 5 wt.%, and more preferably 0.5 to 3 wt.%.

In addition, the composition can suitably comprise a bioactive and or preservative to control bacteria growth. Suitably, the bioactive additive such as for e.g. triclosan, farnesol, ethylhexylglycerin or zinc compounds, such as for e.g. zinc sulphate or zinc coceth laureth. Suitable preservatives are for e.g. is a mixture of sodium methyl paraben, sodium ethyl paraben en phenoxyethanol. The bioactive additive is suitably present in amount of 0.3 to 5 wt%, or preferably, in an amount of 0.5 to 3 wt%, whereas a preservative additive is suitably present up in an amount of 0.2 to1.5 wt%.

The compositions of the invention preferably have a pH in between 4 to 8. In that case, the composition, preferably the emulsion, contains a buffer, which can be selected from the group of consisting of mono-, di-, and tricarboxylic acids and their salts, and substituted mono- and di-carboxylic acids and their salts. Preferably, the buffering agent is lactic acid/lactate and succinic acid/succinate. Most preferably, the buffer is lactic acid/lactate.

A higher initial pH has advantages for the hair removal process. Upon treatment the beard hairs will be softened, due to water-uptake facilitated by the removal of sebum from the outer-surface of the hair and subsequently stretched within the basic emulsion composition environment, allowing a more comfortable, less friction and a closer shave. To this end a preferred embodiment of the invention is a soap free oil-in-water emulsion with the volatile nitrogen base having an pH of from 8 to 12, and which can maintain the pH above 8 for an initial period of time, typically the time during which hair removal is conducted, e.g. during the first 1-5 minutes of the shave. Directly after the shave, it causes to start the skin pH to reduce within 5-10 minutes to a value below 5, as to allow a skin pH within the optimal range for skin barrier recovery, viz. pH 4-5. By providing this aspect of the invention in an embodiment in which the shaving composition is in essence soap-free, a particular benefit can be obtained, in addition to the contribution to skin recovery. This refers to the fact that the skin-drying effect of soap is avoided (and even further improved in the preferred embodiment of a moisturizer composition), with yet the positive aspects of shaving with soap compositions of high pH being retained (see Figure 10).

Typically for a suitable shaving composition, the compositions of the invention, e.g. if in the form of a cream, are capable of attaining a reduced density through application on the skin. Depending on the amount of surfactant, the density after application on the skin will generally be of from 1.04 to 0.70 g/ml.

Preferably, and particularly in the embodiment of an emulsion composition, the compositions in accordance with the present invention can comprise a propellant for promoting foam formation, such as compressed air, dimethyl ether (DME) or a volatile hydrocarbon, preferably with 4 to 6 carbon atoms. This will generally result in a considerably greater reduction in density. Preferably the propellant is of a kind and amount sufficient to provide a reduction in density of the shaving composition of 1.3 to 7 times its original value (0.98 - 1.04 g/ml) after application. Preferably the density of the foam generated through application of the shaving composition on the skin is of from 0.80 to 0.15 g/ml.

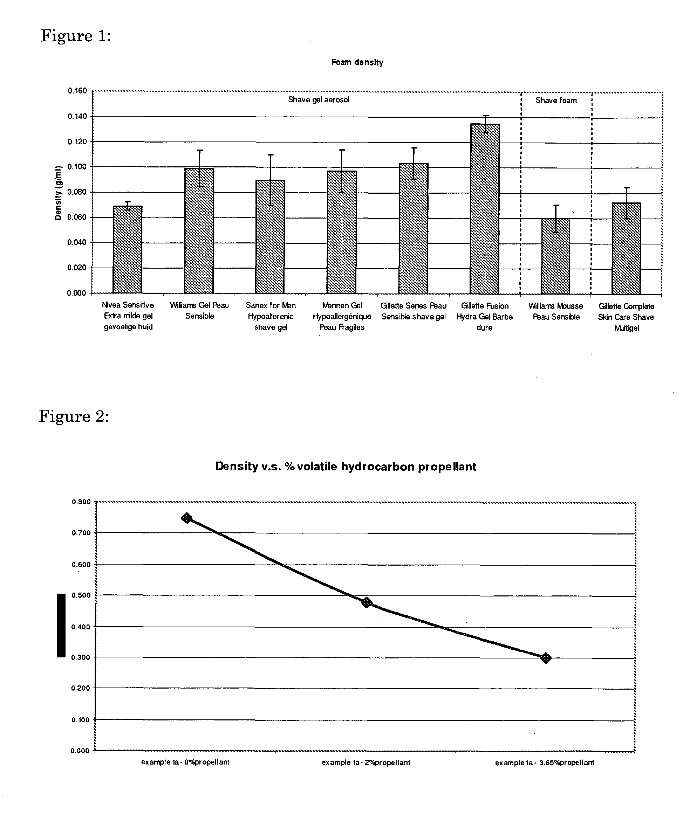

Compared to soap-based and interrupted soap-based post foaming shave gel systems, this decrease in density is lower. These aerosol shaving products show a decrease in density of the shaving composition with a factor of from 7 to 15 times its original value before application on the skin. In fact, the density of these systems decreases from ~1.0 g/ml before application to a density of less than 0.15 g/ml after application on the skin (see Figure 1).

Rather than being a post-foaming shaving composition, which will result in a reduced density after application on the skin, the composition of the invention can also be a foaming composition. In that case the composition is provided with a propellant, particularly a volatile hydrocarbon propellant, of a kind and amount sufficient to provide a reduction in density of the shaving composition of 5 -20 times its original value after application. Typically the density of the foam generated through application of the shaving composition on the skin is of from 0.20 to 0.05 g/ml.

The preferred volatile hydrocarbon has a boiling point below 65°C, preferably below 40°C, and more preferably not below -15°C. Examples of suitable volatile hydrocarbon propellant includes isopentane and isobutane or mixtures of them for the post-foaming shaving compositions and includes (iso)butane and pentane or mixtures of them for the foaming shaving compositions. Hereinafter the term "(iso)butane" serves to indicate isobutane, n-butane, or a mixture thereof

The volatile hydrocarbon, if present, can suitably be present in an amount of maximally 5 wt.%, preferably maximally 4 wt.% and more preferably maximal 3.5 wt.%, of the total emulsion composition. Preferably, the propellant content in the emulsion composition should remain as low as possible to allow the most optimal skin surface contact in combination with an easy visual control to detect unshaven skin surface.

As noted previously, in an embodiment of the invention the emulsion can be provided as a post-foaming shaving composition. Advantageously, due to the evaporation of a volatile hydrocarbon mixture comprising isopentane and isobutane, the density of the post-foaming shaving composition decreases with a factor of from 1.3 to 7 times its original value.

Alternatively, similar effects can be obtained for the foaming shaving composition comprising (iso)butane and propane or mixtures thereof.

A preferred post-foaming propellant system comprises maximally 60 wt.% (iso)butane and minimally 40 wt.% isopentane. More preferably the system comprises 25 to 50 wt.% (iso)butane and 75 to 50 wt.% isopentane. A preferred foaming propellant system comprises at least 70 wt.% (iso)butane and at most 30 wt/% propane, more preferably 80 to 90 wt.% (iso)butane and 20-10 wt.% propane.

Skin-surface contact and improved visuability on the skin are key factors for setting the most optimal decrease in density. A too high decrease in density is not beneficial for an optimal skin-surface contact and can diminish its skin repair effect.

As illustrated by examples 1a, b and c, and figures 2-5, this invention makes it possible to choose an optimal density of the shaving composition. With the generated foam allowing an easy rinse-off from the razor blade, the inventors rate a density reduction by a factor of from 2 to 5 as most preferable for the shaving composition.

The shaving composition preferred according to the present invention serves to optimally address shaving properties, such as support during wet shave with a razor blade, skin barrier recovery, and long-lasting skin moisturizing properties. In addition it also serves to address the desire of a high stability.

In another aspect, the invention pertains to the use of a volatile nitrogen base in the manufacture of an essentially soap-free emulsion for application on the human skin prior to hair-removal, particularly by shaving. More particularly, the invention pertains to the use of a composition comprising 50 to 90 wt.% water, 3 to 20 wt.% oil, 1 to 15 wt.% humectant, up to 20 wt.% of a foam ingredient such as a surfactant or a foam stabilizer, up to 5 % of a structurant such as a thickener, a viscosity enhacer or a rheology modifier and 0.01 to 6 wt.% of a volatile nitrogen base, the percentages being by weight of the entire composition, in the manufacture of an essentially soap-free emulsion for application on the human skin prior to hair-removal, particularly by shaving. For preferred embodiments of the compositions suitable for this use, reference is made to the description of the emulsion components given hereinabove.

The invention also pertains to a shaving composition as described above, for reducing and/or repairing skin damage that has been inflicted by means of a mechanical and/or a chemical process. Also, the invention pertains to the use of a shaving composition as described hereinbefore for enhancing the skin barrier recovery, repair or restoration. Termed otherwise, the invention pertains to the use compositions as substantially described hereinbefore in the manufacture of an essentially soap-free composition, preferably an emulsion, for application on the human skin prior to hair-removal, for the purpose of reducing and/or repairing skin damage that has been inflicted by means of a mechanical and/or a chemical hair-removal process and/or for the purpose of enhancing the skin barrier recovery, repair or restoration.

The compositions, particularly the emulsions, provided serve both as a shaving aid to facilitate preparation and the execution of a wet shave and as skin care after hair removal (i.e. human face or body particularly after shaving, peeling-off hair, or depilation), and preferably are comprised in a shave product.

It will be understood that shaving can apply to any part of the body, including face, head, armpits, legs and the like.

Surprisingly, the composition described in accordance with the invention is very beneficial to the skin, as it allows a rapid repair of the skin barrier function after the skin has been damaged by way of a mechanical and/or chemical process, whereas in addition it moisturizes the skin. More surprisingly, the wetting and stretching conditions during the preparation of beard hair are facilitated such to guarantee a most optimal cutting force and after-shaven skin smoothness.

In contrast with the soap based emulsion compositions that are currently marketed in the market (top 10 products in EU market), the emulsion composition also contributes to an excellent after-shave skin feel and moisturizing properties similar to ordinary aftershave balms, lotions and or moisturisers

It is to be noted that other embodiments than emulsion compositions are included in the invention. E.g. pre-electric shave lotions and polymer-based transparent shave gels. Current market examples of polymer-based transparent shave gels are for e.g. the serie King of Shaves Shave Gel UltraGels or AlphaGels. In addition, the present invention provides an emulsion composition to facilitate a wet-shave using a (electric) razor blade, which comprises the emulsion composition according to the present invention.

Further, the present invention relates also to the use of the compositions of the invention, particularly if in the form of an emulsion composition, as a two-in-one product such as for example a shower&shave product. In that case the product will be able to clean the skin under the shower due to its unique composition comprising also a surfactant and allow the skin to be shaven with the same product.

Further, the present invention also relates to the use of the compositions of the invention, particularly if in the form of an emulsion composition, as a two-in-one product such as for example a deodorant&shave product. In that case the product will be able to deodorize the underarm pit prior to the shave due to its unique composition comprising also a bioactive agent and subsequently allow the skin to be shaven with the same product.

The compositions of the invention, particularly if in the form of an emulsion composition, can be packed in any form of packaging including pots, jars and bottles. More preferably, the emulsion composition can be packed in tin-plated or aluminium aerosol systems or plastic bottles mounted with a valve to allow expansion of the emulsion composition into a foamy texture to occur only out-side the packaging. The emulsion composition of the invention can also be made part of a shaving device by using an integrated dispenser system similar as a conventional lubricant strip, which can be mounted on a disposable razor blade, or a disposable cartridge, which can be mounted on an electronic razor system. During the shave the integrated dispenser system is able to deliver a thin layer comprises an emulsion according to the invention on the skin to facilitate the shave and subsequent to deliver the skin repair properties. This embodiment particularly refers to an emulsion composition which, rather than having foaming characteristics, provides excellent lubricating properties. For this purpose, the emulsion can contain additional water soluble polymers to enhance lubricancy (e.g. various types of polyethylene glycols). This type of integrated packaging is particularly useful with disposable wet-shaving devices, and more particularly with disposable women's wet-shaving devices.

The invention will be further explained hereinafter with reference to the following, non-limiting Figures and Examples.

### Figures 1-9

The figures 1-9 depict graphs that are discussed in Examples 1 and 2. In the figures 1-6 the foam density is on the vertical axis. In the figures 7-9, the respective effect is presented on the vertical axis. The horizontal axis represents the various steps of the test protocol.
**Figure 1****:** foam density of various shave foam and gel aerosol systems
**Figure 2****:** density of shaving composition with 1.7% surfactant (examples 1a) as a function of wt.% of a volatile hydrocarbon propellant mixture
**Figure 3****:** density of shaving composition with 3.6% of a volatile hydrocarbon propellant mixture (examples 1a) as a function of wt.% surfactant
**Figure 4****:** density of shaving composition with 3.6% of a volatile hydrocarbon propellant mixture (examples 1a) as a function of the ratio of isopentane and (iso)butane in the hydrocarbon propellant.
**Figure 5****:** density of shaving composition with 4% of a volatile hydrocarbon propellant mixture (example 1b) as a function of the % product applied during application
**Figure 6****:** Effect of shaving composition (example 1) on skin barrier recovery
**Figure 7****:** Effect of shaving composition (example 1) on skin moisture
**Figure 8****:** Effect of shaving compositions (examples 1 and 2) on skin repair and skin pH properties.
**Figure 9****:** Effect of shaving composition (examples 1 and 2) on skin pH

### Example 1

A shaving composition in accordance with the present invention was prepared as follows. A first mixture was obtained by adding to a separate vessel under stirring 15% cetearyl alcohol, 7% liquid parafin, 1.5% ceteareth-20 and 0.1 % cetearth-12, 0.1% cetyl palmitate, 0.1% glyceryl stearate and 0.1% isohexadecane. A second mixture was obtained by dissolving 7.5% glycerine, 0.03% citric acid and 1% preservative in water. Subsequently, first mixture (oil phase) was mixed with the second mixture (water phase) at 73-77°C, whilst during 10 minutes a homogeniser was applied. The mixture was than cooled down while stirring to 40°C before 0.6 % cyclopentasiloxane, 0.1% and 0.8% fragrance were added. Finally, at 30°C 1.8 % ammonium lactate was added while stirring. After 20 minutes, stirring was stopped and the batch was transferred into a storage container.

### Example 1a

Prior transfer to a post foaming shaving aerosol can, compositions similar to example 1, but with 5% liquid paraffin and 1% cylcopentasiloxane and 0.5% sodium cetearyl sulphate and additionally with either 0 %, 1.7% or 3.3% cocamidopropyl betaine were mixed under pressure with either 2.0, 3.5 or 3.6 wt.% of an isopentane and (iso)butane propellant mixture. The applied ratios isopentane versus (iso)butane were either 75:25, 60:40 or 50:50.

Finally, the mixtures were pushed through the valve into a nylon bag in the aerosol can. Subsequently, the samples were evaluate versus a sample without propellant with respect to the foaming properties.

### Example 1b

A foaming shaving aerosol was filled with 96 wt.% of a shaving composition similar to example 1, but with with 5% liquid paraffin and 1% cylcopentasiloxane and additionally 1.7% cocamidopropyl betaine and 0.5% sodium cetearyl sulphate and subsequently clinched with a for shaving foam dedicated valve. Finally, 4 wt.% (iso)butane and propane propellant mixture was added into the aerosol can through the valve. Prior application, the shaving foam aerosol can was shaken for 30 seconds and the actuator was kept during actuation in a up-right position.

### Example 2 (comparative)

A shaving composition was prepared as follows. A first mixture was obtained by adding to a separate vessel under stirring 15% cetearyl alcohol, 7% liquid parafin, 1.5% ceteareth-20 and 0.1 % cetearth-12, 0.1% cetyl palmitate, 0.1% glyceryl stearate and 0.1% isohexadecane. A second mixture was obtained by dissolving 7.5% glycerine, 0.03% citric acid and 1% preservative in water. Subsequently, first mixture (oil phase) was mixed with the second mixture (water phase) at 73-77°C, whilst during 10 minutes a homogeniser was applied. The mixture was than cooled down while stirring to 40°C before 0.6% cyclopentasiloxane, 0.1% and 0.8% fragrance were added. Finally, after 20 minutes, stirring was stopped and the batch was transferred into a storage container.

### DENSITY MEASUREMENTS

Approx 0.7 gram of shave product was applied on a round glass disc and mixed with approx 0.3 gram of hand-warm water (37°C ). The shave product was mixed for 45 seconds by rotation (2 rounds/second) using one finger. The generated foam was than directly transferred into a small cup of known volume (0.92 ml) and subsequently, the weight was measured. Precaution was taken to fill the cup completely without pressing the foam. Average of 5 readings was taken, where each reading was obtained by making a fresh sample of foam.

Foam density measurements of the shave foam aerosol product was measured directly after discharge of the foam from the aerosol can.

### TESTING SKIN RECOVERY AND SKIN pH

The shaving composition in accordance with the present invention has been used to stimulate to repair the barrier of the tape-stripped skin of 12 subjects (individuals). A subgroup of 8 subjects participated during the test which were not able to acidify their skin in relatively short notice after an alkaline treatment like washing with soap (so called slow-responders).

### TAPE-STRIP PROTOCOL

Three fields (3x3 cm) on the volar side of the forearms were tape stripped with Pritt Sellotape Diamond ultra clear (Henkel) until Trans Epidermal Water Loss (TEWL) increase was in the range 15-25 g.m⁻².h⁻¹. The following procedure was used:
- A piece of tape was applied at skin;
- A preheated stainless steel weight (1000g, 64g/cm²; T=34°C) was applied for five seconds at the taped skin;
- The tape was removed by jerking parallel to the skin (as fast as possible);
- This procedure was repeated 15-30 times.

### RECOVERY PERIOD

The barrier repair of the tape-stripped human skin was monitored during a period of nine days, both in respect of skin treated twice a day with the test product as well as untreated skin. During this period, the skin was soap-washed twice daily. This monitoring was done by measuring the TEWL values - values that are an excellent indicator for the quality of the skin's barrier.

### SOAP WASHING PROCEDURE

The inner sides of the forearms were washed after the tape stripping procedure on the evening of the first day, and subsequently on every morning and evening up to and including the morning of the ninth day. During the washing procedure the following protocol was used:
- The forearms were wetted with lukewarm tap water;
- During two minutes the forearms were washed by the subject with a 19% solution of natural soap bar (rubbing now and then);
- The forearms were then rinsed with tap water during about 15 seconds.
- The forearms were subsequently dried by means of dapping with a towel.

### PRODUCT APPLICATION PROTOCOL

The shaving compositions (examples 1 and 2) as described hereinbefore was applied at the tape-stripped skin by the subjects themselves. About 2 mg per cm² was applied every time. On the first day the composition was applied onto the skin after the last tape-stripping and after each soap washing, whereas on the second day up to and including the morning of the ninth day the composition was applied onto the skin after each soap washing.

### BIOPHYSICAL ASSESSMENTS

The TEWL values and skin pH values were then measured of the parts of skin treated as described hereinbefore. On the first day the values were measured before the treatment was started (baseline measurement), during the tape-stripping procedure (only TEWL values, data not shown), and 15 minutes after the soap washing treatment (start recovery measurement). Subsequently, the values were measured on the third, seventh and ninth day four hours after the shaving compositions have been applied onto the skin. The TEWL values were measured by means of a Tewameter^{®} TM300 (Courage + Khazaka), the skin moisture was measured by means of a Cornemeter^{®} CM825, whereas the pH value of the skin was measured by means of a Skin-pH-Meter^{®} PH 905 (Courage + Khazaka) 4 hours after application of the hair removal emulsion composition. The subjects were 10 healthy volunteers of either sex who were aged between 18 and 65 years. The following conditions were applied during the measurements: a temperature of 22° C (standard deviation of 1°C); a relative humidity of 45% (standard deviation of 5%); an equilibration period of 30 minutes. In the statistical analysis a two-tailed Paired Student's t-test was applied and a critical p-value (probability-value) for significance of 0.05 was used. In the data obtained the recovery is expressed as (1 - "TEWL increase" / "initial TEWL increase")*100%, whereas the TEWL increase is defined as the TEWL value of tape stripped skin minus the TEWL value of the intact skin. In other words, both the three tape-stripped skin fields as well as the intact (soap-washed) skin field were measured.

### RESULTS

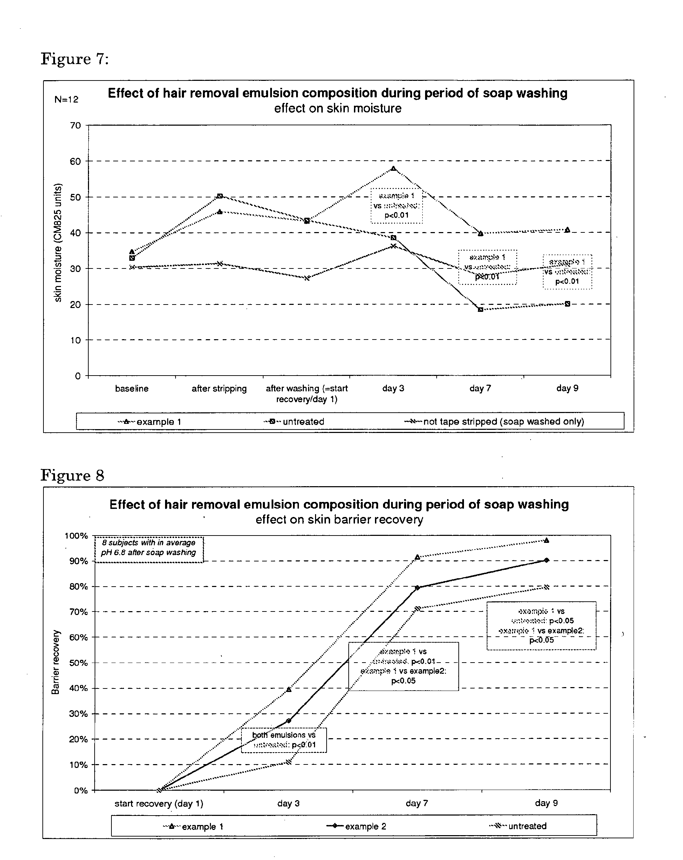

From the results shown in Figure 1, it will be clear that the density of the generated foam from shave foam and shave gel aerosol products purchased on the EU/USA market is below 0.15 g/ml. In addition, the use of the post foaming shaving composition in accordance with the present invention (example 1a) show a significant higher density (Figures 2 and 3). Alternative, the foaming shaving composition in accordance with the present invention (example 1b) show similar density values of 0.08 - 0.12 mg/ml (Figure 5). The density can be influenced by changing the composition of the emulsion or by changing the amount of propellant mixture or the composition of the propellant mixture used to deliver the most optimal layer (Figures 4 and 5). From the results shown in Figure 6, it will be clear that repeated application of the present shaving composition on tape-stripped human skin resulted in a significantly quicker recovery of the barrier of the skin compared to undamaged skin. In addition, the use of the composition in accordance with the present invention and described in example 1 also resulted in a significant improvement of the skin moisture (Figure 7) compared to undamaged skin. From the results shown in Figures 8-9, it will be clear that the skin repair properties of the emulsion composition with a volatile nitrogen base (example 1) is superior versus the skin repair properties of the emulsion composition without the volatile nitrogen base (example 2) for subjects which are not able to acidify their skin in relative short notice after an alkaline treatment. Also the skin pH and skin moisturising properties where significantly better for the shaving composition according example 1, thus with the volatile nitrogen base, than the shaving composition according to example 2.

### Figures 10-11

The figures 10-11 depict graphs that are discussed in Example 3a-3f and example 4. In each Figure, the respective effect is presented on the vertical axis. The horizontal axis represents the various steps of the test protocol discussed in the Example.

**Figure 10****:** Effect of shaving composition (examples 3a-3d and 4) on skin pH during and directly after the application.

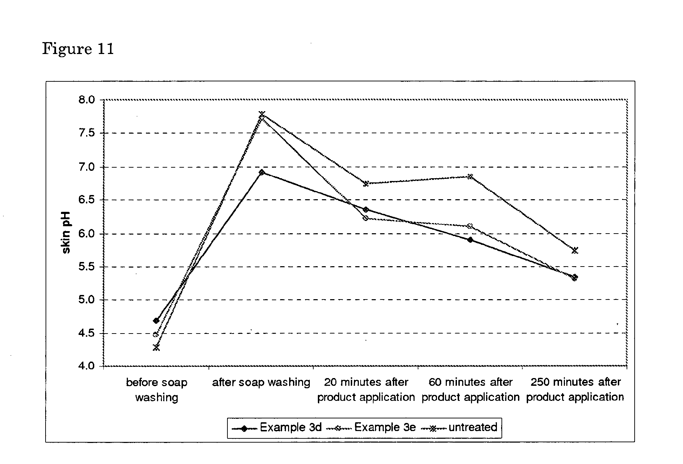

**Figure 11****:** Effect of shaving composition (examples 3e-3f) on skin pH during and directly after the application.

### Example 3a

Shaving composition 3a, in accordance with the present invention, was prepared as follows: A first mixture was obtained by adding to a separate vessel under stirring 14% cetearyl alcohol, 6% liquid parafin, 1.4% ceteareth-20 and 0.1 % cetearth-12, 0.1% cetyl palmitate, 0.1% glyceryl stearate and 0.1% isohexadecane. A second mixture was obtained by dissolving 7% glycerine, 1% triethanolamine and 0.1% poatassium hydroxide and 0.6% preservative in water. Subsequently, first mixture (oil phase) was mixed with the second mixture (water phase) at 73-77°C, whilst during 10 minutes a homogeniser was applied. The mixture was than cooled down while stirring to 40°C before 0.6 % cyclopentasiloxane, 0.1% and 0.8% fragrance were added. Finally, at 30°C 1.4% ammonium lactate was added while stirring. After 20 minutes, stirring was stopped and the batch was transferred into a storage container. A pH of 8.2 was recorded one day after the production of the samples and prior to the product application test

### Example 3b

A first mixture was obtained by adding to a separate vessel under stirring 15% cetearyl alcohol, 7% liquid parafin, 1.5% ceteareth-20 and 0.1 % cetearth-12, 0.1% cetyl palmitate, 0.1% glyceryl stearate and 0.1% isohexadecane. A second mixture was obtained by dissolving 7% glycerine, 0.4% potassium hydroxide and 0.6% preservative in water. Subsequently, first mixture (oil phase) was mixed with the second mixture (water phase) at 73-77°C, whilst during 10 minutes a homogeniser was applied. The mixture was than cooled down while stirring to 40°C before 0.6 % cyclopentasiloxane, 0.1% and 0.8% fragrance were added. Finally, at 30°C 1.4% ammonium lactate was added while stirring. After 20 minutes, stirring was stopped and the batch was transferred into a storage container. A pH of 8.9 was recorded one day after the production of the samples and prior to the product application test.

### Example 3c

A first mixture was obtained by adding to a separate vessel under stirring 14% cetearyl alcohol, 6% liquid parafin, 1.4% ceteareth-20 and 0.1 % cetearth-12, 0.1% cetyl palmitate, 0.1% glyceryl stearate and 0.1% isohexadecane. A second mixture was obtained by dissolving 7% glycerine, 0.7% glycine and 0.3% potassium hydroxide and 1% preservative in water. Subsequently, first mixture (oil phase) was mixed with the second mixture (water phase) at 73-77°C, whilst during 10 minutes a homogeniser was applied. The mixture was than cooled down while stirring to 40°C before 0.6 % cyclopentasiloxane, 0.1% and 0.8% fragrance were added. Finally, at 30°C 1.2% ammonium lactate was added while stirring. After 20 minutes, stirring was stopped and the batch was transferred into a storage container. A pH of 8.1 was recorded one day after the production of the samples and prior to the product application test.

### Example 3d (comparative)

Shaving composition 3d was prepared as follows. A first mixture was obtained by adding to a separate vessel under stirring 15% cetearyl alcohol, 7% liquid parafin, 1.5% ceteareth-20, 0.1% cetearth-12, 0.1% cetyl palmitate, 0.1% glyceryl monostearate and 0.1% isohexadecane. A second mixture was obtained by dissolving 7.5% glycerine, 0.03% citric acid and 1% preservative in water. Subsequently, first mixture (oil phase) was mixed with the second mixture (water phase) at 73-77 °C, whilst during 10 minutes a homogeniser was applied. The mixture was than cooled down while stirring to 49°C before 0.1% panthenol, 0.6% cyclopentasiloxane and 0.8% fragrance were added. Subsequently, at 38°C again a homogeniser was applied during 1 minute. Finally, the stirring was stopped until a homogeneous mixture was obtained. A pH of 4.8 was recorded for examples 3d one day after the production of the samples and prior to the product application test.

### Example 3g

Shaving composition 3g was prepared as follows. A first mixture was obtained by adding to a separate vessel under stirring 15% cetearyl alcohol, 7% liquid parafin, 1.5% ceteareth-20, 0.1% cetearth-12, 0.1% cetyl palmitate, 0.1% glyceryl stearate and 0.1% isohexadecane. A second mixture was obtained by dissolving 7.5% glycerine, 0.5% preservative in water. Subsequently, first mixture (oil phase) was mixed with the second mixture (water phase) at 73-77 °C, whilst during 10 minutes a homogeniser was applied. The mixture was than cooled down while stirring to 45°C before 0.1% panthenol, 0.6% cyclopentasiloxane, 0.8% fragrance,0.1% sodium lactate, 0.4% lactic acid and 0.6% ammonium lactate were added. Subsequently, at 38°C again a homogeniser was applied during 5 minutes. The stirring was stopped until a homogeneous mixture was obtained. A pH 4.7 was measured for example 3g one day after the production of the sample and prior to the product application test.

### Example 4 (comparative)

A post foaming soap base shave gel composition was prepared as follows. A first mixture was obtained by adding to the main vessel under stirring water, 7.5% triethanolamine, 0.5% PEG-4 rapeseediamine. The temperature of this mixture was heated 75-80 °C. Than a second mixture was added, which was obtained by mixing 1.75% sorbitol with 0.3% hydroxyethylcellulose and 0.1% PEG-2M. Subsequently, the wax mixture was added at 75-80 °C, which was obtained by pre-heating a mixture of 2.7% sorbitan plamitate, 14% palmitic acid and 0.05% BHT while stirring in a separate vessel. The mixture was homoginized for 10 minutes at 75-80 °C under vacuum. Than the mixture was slowly cooled down till 29°C while stirring under vacuum. At 35°C, 0.5% fragrance was added, followed by 2% ammonium lactate at 30°C. The stirring was stopped until a homogeneous mixture was obtained.

### Example 3e (comparative)

Shaving composition 3e was prepared as follows. A first mixture was obtained by adding to a separate vessel under stirring 15% cetearyl alcohol, 7% liquid parafin, 1.5% ceteareth-20, 0.1% cetearth-12, 0.1% cetyl palmitate, 0.1% glyceryl stearate and 0.1% isohexadecane. A second mixture was obtained by dissolving 7.5% glycerine, 0.5% preservative in water. Subsequently, first mixture (oil phase) was mixed with the second mixture (water phase) at 73-77 °C, whilst during 10 minutes a homogeniser was applied. The mixture was than cooled down while stirring to 45°C before 0.1% panthenol, 0.6% cyclopentasiloxane, 0.8% fragrance,0.7% sodium lactate and 0.4% lactic acid were added. Subsequently, at 38°C again a homogeniser was applied during 5 minutes. The stirring was stopped until a homogeneous mixture was obtained. A pH 4.6 was measured for example 3e one day after the production of the sample and prior to the product application test.

### Example 3f

Shaving composition 3f in accordance with the present invention was prepared as follows. A first mixture was obtained by adding to a separate vessel under stirring 10% cetearyl alcohol, 5% liquid parafin, 1.5% ceteareth-20, 0.1% cetearth-12, 0.7% palmitic acid. A second mixture was obtained by dissolving 5% glycerine, 3% cocamidopropyl betaine, 1% preservative and 1.3% triethanolamine in water. Subsequently, first mixture (oil phase) was mixed with the second mixture (water phase) at 75-80 °C, whilst during 10 minutes a homogeniser was applied. The mixture was than cooled down while stirring to 45°C before 1% cyclopentasiloxane and 0.5% fragrance were added. Subsequently, at 38°C again a homogeniser was applied during 1 minute. Finally, at a temperature less than 30°C, 1.8% ammonium lactate was added while stirring. The stirring was stopped until a homogeneous mixture was obtained. A pH 8.0 was measured for example 3f one day after the production of the sample and prior to the product application test.

A pH 8.2 was measured for example 4 three days after the production of the sample and prior to the product application test.

### PRODUCT APPLICATION AND BIOPHYSICAL ASSESMENT PROTOCOL

The pH value of the skin was measured by means of a Skin-pH-Meter^{®} PH 905 (Courage + Khazaka). The subjects were healthy volunteers of either sex who were aged between 18 and 65 years. The following conditions were applied during the measurements: a temperature of 22° C (standard deviation of 1°C); a relative humidity of 45% (standard deviation of 5%); an equilibration period of 30 minutes.

At start of the test, the vollar sides of the forearms were washed with soap for 20 seconds. Subsequently, the skin was rinsed with water to remove the soap. 1 minute later the pH of the various test skin fields were measured. Once these measurements were completed approximately 2 mg per cm² of the shaving compositions 3a-3f and 4 were applied. pH-assessments of the various test skin fields were recorded 5, 10, 20, 40 and 60 minutes after application.

### RESULTS

From the results shown in Figures 10-11, it will be clear that only application of shaving compositions with a volatile nitrogen base will lead to a pH reduction on skin (see examples 3a-3c and 3g versus ex 3d-3e). The effect is established with 5 to 10 minutes after application. Shaving compositions with soap (> 2%) will not give this beneficial effect and the skin pH remains for a very long time at a pH > 6 after the application of the product (see examples 3f and 4).

## Claims

1. The use of an essentially soap-free composition, having maximally 2 wt.% of soap, in shaving the skin, the composition comprising 50-90 wt.% water, 1-15 wt.% humectant, 0.1 to 20 wt.% of a surfactant and/or a foam stabilizer, up to 5 wt.% of a structurant such as a thickener, a viscosity enhancer or a rheology modifier and 0.01-6 wt.% of a volatile nitrogen base, the percentages being by weight of the entire composition.

2. A use according to claim 1, wherein the composition is in the form of an emulsion comprising 50-90 wt.% water, 3-20 wt.% of a mixture of hydrocarbon oils, vegetable oils, synthetic oils and silicon oils, 1-15 wt.% humectant, up to 20 wt.% of a surfactant and/or a foam stabilizer, up to 5 wt.% of a structurant such as a thickener, a viscosity enhacer or a rheology modifier and 0.01-6 wt.% of a volatile nitrogen base, the percentages being by weight of the entire composition.

3. A use according to claim 1, wherein the composition comprises an effective amount of a propellant.

4. A use according to claim 3, wherein the composition comprises 50-75 wt.% water, 7-18 wt.% of a mixture of hydrocarbon and silicon oils, 1-15 wt.% humectant, up to 20 wt.% surfactant and/or a foam stabilizer, up to 5 wt.% of a structurant such as a thickener, a viscosity enhacer or a rheology modifier, 0.01-6 wt.% of a volatile nitrogen base, and 1-5 wt.% of a volatile hydrocarbon propellant mixture.

5. A use according to claim 4, wherein the volatile hydrocarbon propellant comprises a mixture selected from the group consisting of (a) maximally 60 wt.% (iso)butane and minimally 40 wt.% isopentane, and (b) minimally 75 wt.% (iso)butane and maximally 25 wt.% propane.

6. A use according to any one of the claims, wherein the composition has a pH of from 4 to 12 and wherein the volatile nitrogen base reduces the skin pH to a targeted skin pH of in between 4 and 5 within 5-10 minutes after completion of the shave.

7. A use according to any one of the preceding claims, wherein the volatile nitrogen base is selected from the group consisting of ammonia, mono-ethanol amine, alkyl amines of which the alkyl group comprises 1-4 carbon atoms, and mixtures thereof.

8. A use according to any one of the preceding claims, comprising the volatile nitrogen base in a concentration of from 0.01 - 6 wt.% of the total emulsion composition.

9. A use according to any one of the preceding claims, wherein the volatile nitrogen base is selected from the group consisting of ammonium chloride, ammonium sulphate, ammonium lactate, and mixtures thereof.

10. A use according to any one of the preceding claims, wherein the composition has a pH of from 4 to 8, and wherein the composition contains a lactic acid/lactate buffer or succinic acid/succinate buffer.

11. A use according any one of the preceding claims, wherein the composition has a pH of from 8 to 12, and wherein the composition contains component(s) selected from the group consisting of triethanolamine, ethanolamine, glycine and sodium carbonate.

12. The use according to any one of the preceding claims, wherein the composition has a bifold use selected from the group consisting of (a) shaving and aftershaving, (b) shaving and deodorizing, wherein the composition preferably comprises 0.1-5 wt.% of a bioactive ingredient, (c) showering and shaving.

13. An essentially soap-free shaving composition, having maximally 2 wt.% of soap, the composition comprising 50-90 wt.% water, 1-15 wt.% humectant, 0.1 to 20 wt.% of a surfactant and/or a foam stabilizer, up to 5 wt.% of a structurant such as a thickener, a viscosity enhacer or a rheology modifier and 0.01-6 wt.% of a volatile nitrogen base, and an effective amount of a propellant the percentages being by weight of the entire composition.

14. An essentially soap-free shaving composition, having maximally 2 wt.% of soap, comprising 50-90 wt.% water, 1-15 wt.% humectant, 0.1 to 20 wt.% of a surfactant and/or a foam stabilizer, up to 5 wt.% of a structurant such as a thickener, a viscosity enhacer or a rheology modifier and 0.01-6 wt.% of a volatile nitrogen base selected from the group consisting of ammonium chloride, ammonium sulphate, ammonium lactate, and mixtures thereof, the percentages being by weight of the entire composition.

15. A composition according to claim 13 or 14, comprising the components as defined in any one of the claims 1-12.

## Patentansprüche

1. Verwendung einer im wesentlichen seifenfreien Zusammensetzung mit maximal 2 Gew.-% Seife beim Rasieren der Haut, wobei die Zusammensetzung 50 bis 90 Gew.-% Wasser, 1 bis 15 Gew.-% Feuchthaltemittel, 0,1 bis 20 Gew.-% eines Tensides und/oder eines Schaumstabilisators, bis zu 5 Gew.-% eines Strukturbildners wie von einem Verdicker, einem Viskositätsverstärker oder einem Rheologie-Modifizierer und 0,01 bis 6 Gew.-% einer flüchtigen Stickstoffbase umfasst, wobei die Gewichtsprozente sich auf das Gewicht der gesamten Zusammensetzung beziehen.

2. Verwendung gemäß Anspruch 1, wobei die Zusammensetzung in der Form einer Emulsion umfassend 50 bis 90 Gew.-% Wasser, 3 bis 20 Gew.-% einer Mischung von Mineralölen, Pflanzenölen, synthetischen Ölen und Siliconölen, 1 bis 15 Gew.-% Feuchthaltemittel, bis zu 20 Gew.-% eines Tensides und/oder eines Schaumstabilisators, bis zu 5 Gew.-% eines Strukturbildners wie von einem Verdicker, einem Viskositätsverstärker oder einem Rheologie-Modifizierer und 0,01 bis 6 Gew.-% einer flüchtigen Stickstoffbase vorliegt, wobei die Gewichtsprozente sich auf das Gewicht der gesamten Zusammensetzung beziehen.

3. Verwendung gemäß Anspruch 1, wobei die Zusammensetzung eine wirksame Menge eines Treibmittels umfasst.

4. Verwendung gemäß Anspruch 3, wobei die Zusammensetzung 50 bis 75 Gew.-% Wasser, 7 bis 18 Gew.-% einer Mischung von Mineralölen und Siliconölen, 1 bis 15 Gew.-% Feuchthaltemittel, bis zu 20 Gew.-% Tensid und/oder Schaumstabilisator, bis zu 5 Gew.-% eines Strukturbildners wie von einem Verdicker, einem Viskositätsverstärker oder einem Rheologie-Modifizierer, 0,01 bis 6 Gew.-% einer flüchtigen Stickstoffbase und 1 bis 5 Gew.-% einer flüchtigen Kohlenwasserstoff-Treibmittelmischung umfasst.

5. Verwendung gemäß Anspruch 4, wobei das flüchtige Kohlenwasserstoff-Treibmittel eine Mischung ausgewählt aus der Gruppe bestehend aus (a) maximal 60 Gew.-% Isobutan und mindestens 40 Gew.-% Isopentan, und (b) mindestens 75 Gew.-% Isobutan und maximal 25 Gew.-% Propan umfasst.

6. Verwendung gemäß irgendeinem der Ansprüche, wobei die Zusammensetzung einen pH-Wert von 4 bis 12 hat und die flüchtige Stickstoffbase innerhalb von 5 bis 10 Minuten nach Vollendung der Rasur den pH-Wert der Haut zu einem angestrebten Haut-pH-Wert zwischen 4 und 5 verringert.

7. Verwendung gemäß irgendeinem der vorhergehenden Ansprüche, wobei die flüchtige Stickstoffbase aus der Gruppe bestehend aus Ammoniak, Monoethanolamin, Alkylaminen deren Alkylgruppe 1 bis 4 Kohlenstoffatome umfasst und Mischungen davon ausgewählt ist.

8. Verwendung gemäß irgendeinem der vorhergehenden Ansprüche, umfassend die flüchtige Stickstoffbase in einer Konzentration von 0,01 bis 6 Gew.-% der gesamten Emulsionszusammensetzung.

9. Verwendung gemäß irgendeinem der vorhergehenden Ansprüche, wobei die flüchtige Stickstoffbase aus der Gruppe bestehend aus Ammoniumchlorid, Ammoniumsulfat, Ammoniumlactat und Mischungen davon ausgewählt ist.

10. Verwendung gemäß irgendeinem der vorhergehenden Ansprüche, wobei die Zusammensetzung einen pH-Wert von 4 bis 8 hat und wobei die Zusammensetzung einen Milchsäure/Lactat-Puffer oder Bernsteinsäure/Succinat-Puffer enthält.

11. Verwendung gemäß irgendeinem der vorhergehenden Ansprüche, wobei die Zusammensetzung einen pH-Wert von 8 bis 12 hat und wobei die Zusammensetzung Bestandteile ausgewählt aus der Gruppe bestehend aus Triethanolamin, Ethanolamin, Glycin und Natriumcarbonat enthält.

12. Verwendung gemäß irgendeinem der vorhergehenden Ansprüche, wobei die Zusammensetzung eine zweifache Verwendung ausgewählt aus der Gruppe bestehend aus (a) Rasieren und Aftershave, (b) Rasieren und Desodorieren, wobei die Zusammensetzung vorzugsweise 0,1 bis 5 Gew.-% einer bioaktiven Zutat umfasst, (c) Duschen und Rasieren hat.

13. Im wesentlichen seifenfreie Zusammensetzung mit maximal 2 Gew.-% Seife, wobei die Zusammensetzung 50 bis 90 Gew.-% Wasser, 1 bis 15 Gew.-% Feuchthaltemittel, 0,1 bis 20 Gew.-% eines Tensides und/oder eines Schaumstabilisators, bis zu 5 Gew.-% eines Strukturbildners wie von einem Verdicker, einem Viskositätsverstärker oder einem Rheologie-Modifizierer und 0,01 bis 6 Gew.-% einer flüchtigen Stickstoffbase sowie eine wirksame Menge eines Treibmittels umfasst, wobei die Gewichtsprozente sich auf das Gewicht der gesamten Zusammensetzung beziehen.

14. Im wesentlichen seifenfreie Zusammensetzung mit maximal 2 Gew.-% Seife, umfassend 50 bis 90 Gew.-% Wasser, 1 bis 15 Gew.-% Feuchthaltemittel, 0,1 bis 20 Gew.-% eines Tensides und/oder eines Schaumstabilisators, bis zu 5 Gew.-% eines Strukturbildners wie von einem Verdicker, einem Viskositätsverstärker oder einem Rheologie-Modifizierer und 0,01 bis 6 Gew.-% einer flüchtigen Stickstoffbase ausgewählt der Gruppe bestehend aus Ammoniumchlorid, Ammoniumsulfat, Ammoniumlactat und Mischungen davon umfasst, wobei die Gewichtsprozente sich auf das Gewicht der gesamten Zusammensetzung beziehen.

15. Zusammensetzung gemäß Anspruch 13 oder 14, umfassend die Bestandteile wie in irgendeinem der Ansprüche 1 bis 12 definiert.

## Revendications

1. Utilisation d'une composition essentiellement exempte de savon, présentant au maximum 2 % en poids de savon, dans un rasage de la peau, la composition comprenant 50-90 % en poids d'eau, 1-15 % en poids d'humectant, 0,1 à 20 % en poids d'un tensioactif et/ou d'un stabilisant de mousse, jusqu'à 5 % en poids d'un structurant, tel qu'un épaississant, un promoteur de viscosité ou un modificateur de rhéologie et 0,01-6 % en poids d'une base d'azote volatile, les pourcentages étant en poids de la composition totale.

2. Utilisation selon la revendication 1, dans laquelle la composition est dans la forme d'une émulsion comprenant 50-90 % en poids d'eau, 3-20 % en poids d'un mélange d'huiles hydrocarbonées, d'huiles végétales, d'huiles synthétiques et d'huiles de silicium, 1-15 % en poids d'humectant, jusqu'à 20 % en poids d'un tensioactif et/ou d'un stabilisant de mousse, jusqu'à 5 % en poids d'un structurant, tel qu'un épaississant, un promoteur de viscosité ou un modificateur de rhéologie et 0,01-6 % en poids d'une base d'azote volatile, les pourcentages étant en poids de la composition totale.

3. Utilisation selon la revendication 1, dans laquelle la composition comprend une quantité efficace d'un propulseur.

4. Utilisation selon la revendication 3, dans laquelle la composition comprend 50-75 % en poids d'eau, 7-18 % en poids d'un mélange d'huiles hydrocarbonées et de silicium, 1-15 % en poids d'humectant, jusqu'à 20 % en poids de tensioactif et/ou d'un stabilisant de mousse, jusqu'à 5 % en poids d'un structurant, tel qu'un épaississant, un promoteur de viscosité ou un modificateur de rhéologie, 0,01-6 % en poids d'une base d'azote volatile, et 1-5 % en poids d'un mélange de propulseur hydrocarboné volatil.

5. Utilisation selon la revendication 4, dans laquelle le propulseur hydrocarboné volatil comprend un mélange choisi dans le groupe constitué de (a) au maximum 60 % en poids d'(iso)butane et au minimum 40 % en poids d'isopentane, et (b) au minimum 75 % en poids d'(iso)butane et au maximum 25 % en poids de propane.

6. Utilisation selon l'une quelconque des revendications, dans laquelle la composition présente un pH de 4 à 12 et dans laquelle la base d'azote volatile réduit le pH de la peau à un pH de peau ciblé entre 4 et 5 en 5-10 min après l'achèvement du rasage.

7. Utilisation selon l'une quelconque des revendications précédentes, dans laquelle la base d'azote volatile est choisie dans le groupe constitué d'ammoniac, mono-éthanolamine, alkylamines dont le groupe alkyle comprend 1-4 atomes de carbone, et mélanges de ceux-ci.

8. Utilisation selon l'une quelconque des revendications précédentes, comprenant la base d'azote volatile dans une concentration de 0,01-6 % en poids de la composition d'émulsion totale.

9. Utilisation selon l'une quelconque des revendications précédentes, dans laquelle la base d'azote volatile est choisie dans le groupe constitué de chlorure d'ammonium, sulfate d'ammonium, lactate d'ammonium, et mélanges de ceux-ci.

10. Utilisation selon l'une quelconque des revendications précédentes, dans laquelle la composition présente un pH de 4 à 8, et dans laquelle la composition contient un tampon d'acide lactique/lactate ou un tampon d'acide succinique/succinate.

11. Utilisation selon l'une quelconque des revendications précédentes, dans laquelle la composition présente un pH de 8 à 12, et dans laquelle la composition contient un(des) constituant(s) choisi(s) dans le groupe constitué de triéthanolamine, éthanolamine, glycine et carbonate de sodium.

12. Utilisation selon l'une quelconque des revendications précédentes, dans laquelle la composition présente une utilisation double choisie dans le groupe constitué de (a) rasage et après-rasage, (b) rasage et désodorisation, dans laquelle la composition comprend de préférence 0,1-5 % en poids d'un ingrédient bioactif, (c) douche et rasage.

13. Composition de rasage essentiellement exempte de savon, présentant au maximum 2 % en poids de savon, la composition comprenant 50-90 % en poids d'eau, 1-15 % en poids d'humectant, 0,1 à 20 % en poids d'un tensioactif et/ou d'un stabilisant de mousse, jusqu'à 5 % en poids d'un structurant, tel qu'un épaississant, un promoteur de viscosité et un modificateur de rhéologie et 0,01-6 % en poids d'une base d'azote volatile, et une quantité efficace d'un propulseur, les pourcentages étant en poids de la composition totale.

14. Composition de rasage essentiellement exempte de savon, présentant un maximum de 2 % en poids de savon, comprenant 50-90 % en poids d'eau, 1-15 % en poids d'humectant, 0,1 à 20 % en poids d'un tensioactif et/ou d'un stabilisant de mousse, jusqu'à 5 % en poids d'un structurant, tel qu'un épaississant, un promoteur de viscosité ou un modificateur de rhéologie et 0,01-6 % en poids d'une base d'azote volatile choisie dans le groupe constitué de chlorure d'ammonium, sulfate d'ammonium, lactate d'ammonium, et mélanges de ceux-ci, les pourcentages étant en poids de la composition totale.

15. Composition selon la revendication 13 ou 14, comprenant les constituants comme définis dans l'une quelconque des revendications 1-12.
